# EUROPÄISCHE PATENTSCHRIFT

(11) **EP 2 155 386 B1**
(45) Veröffentlichungstag und Bekanntmachung des Hinweises auf die Patenterteilung: **22.02.2012**
(21) Anmeldenummer: 08750014.6
(22) Anmeldetag: 05.05.2008
(51) Int. Cl.: B01J 23/89, C07C 209/36

(54) **VERFAHREN ZUR HERSTELLUNG VON AMINEN**
METHOD FOR PRODUCING AMINES
PROCÉDÉ POUR LA PRÉPARATION D'AMINES

(30) Priorität: 10.05.2007 EP 07107893
(43) Veröffentlichungstag der Anmeldung: 24.02.2010
(73) Patentinhaber: BASF SE, 67056 Ludwigshafen (DE)
(72) Erfinder: COELHO TSOU, Joana, 69120 Heidelberg (DE); SCHWAB, Ekkehard, 67434 Neustadt (DE); KUBANEK, Petr, 68163 Mannheim (DE); MACKENROTH, Wolfgang, B-3080 Tervuren (BE); OEHLENSCHLÄGER, Steffen, 67063 Ludwigshafen (DE); VOSS, Hartwig, 67227 Frankenthal (DE); NETO, Samuel, 01099 Dresden (DE)
(86) Internationale Anmeldenummer: PCT/EP2008/055447
(87) Internationale Veröffentlichungsnummer: WO 2008/138784

(56) Entgegenhaltungen:
- WO-A-2006/134403
- US-A- 3 781 227
- DATABASE WPI Week 199826 Thomson Scientific, London, GB; AN 1998-295624 XP002494521 & RU 2 095 136 C1 (MONOMERS RES DES INST) 10. November 1997 (1997-11-10)

## Beschreibung

Die Erfindung betrifft ein Verfahren zur Herstellung von Aminen durch katalytische Hydrierung der entsprechenden Nitroverbindungen sowie neue Katalysatoren zur Durchführung des Verfahrens.

Die Herstellung von Aminen, insbesondere von aromatischen Mono-, Di- und/oder Polyaminen durch katalytische Hydrierung der entsprechenden Mono-, Di- und/oder Polynitroverbindungen ist seit langem bekannt und vielfach in der Literatur beschrieben. Ein in der Technik häufig eingesetztes aromatisches Amin ist das Toluylendiamin (TDA), das zu Toluylendiisocyanat weiterverarbeitet werden kann und durch Hydrierung von Dinitrotoluol (DNT) hergestellt wird. Ein Problem bei der Hydrierung von DNT ist die verstärkte Bildung von Nebenprodukten, neben Leichtsiedern, zumeist desaminierte und kernhydrierte Produkte, kommen häufig höhermolekulare oder teerartige Produkte vor, die neben der Verringerung der Ausbeute des Verfahrens auch zu einer vorzeitigen Desaktivierung des Katalysators führen können.

Als Hydrierkatalysatoren werden, wie beispielsweise in EP-A-0 124 010 beschrieben, häufig Metalle der VIII. Nebengruppe des Periodensystems, insbesondere Raney-Eisen, Raney-Kobalt und Raney-Nickel, eingesetzt.

Häufig werden für die Hydrierung von Nitroaromaten auch Katalysatoren eingesetzt, die Edelmetalle, insbesondere Palladium, oder auch Platin, enthalten. Bekannt sind hierbei auch Katalysatoren, die Platin und Nickel enthalten.

So beschreibt US 3,127,356 ein Verfahren zur Herstellung von Hydrierkatalysatoren, die für die Hydrierung von DNT zu TDA eingesetzt werden können. Die Katalysatoren enthalten einen Träger, eine oleophile Kohlenstoffkomponente, wie zum Beispiel ein Ruß, auf der die Metalle aufgebracht sind. Dabei liegt das Nickel im Katalysator als Oxid oder Hydroxid vor.

US 5,214,212 beschreibt ein Verfahren zur Kernhydrierung von aromatischen Aminen. Als Katalysator wird ein Edelmetallkatalysator eingesetzt, der zusätzlich mit weiteren Metallen, unter anderem Nickel, dotiert sein kann. Als Edelmetall kann Platin im Gemisch mit anderen Edelmetallen eingesetzt werden. Dabei liegen die Edelmetalle im Katalysator als Metalle und die dotierten Metalle in Form von Salzen vor.

In DE 39 28 329 wird ein Verfahren zur Herstellung von chlorsubstituierten aromatischen Aminen aus den entsprechenden Nitroverbindungen beschrieben. Der bei diesem Verfahren eingesetzte Katalysator besteht aus Aktivkohle als Träger, auf die Platin und ein weiteres Metall, insbesondere Nickel, aufgebracht sind.

In EP 595 124 wird ein Verfahren zur Herstellung von chlorsubstituierten aromatischen Aminen aus den entsprechenden Nitroverbindungen beschrieben. Der verwendete Katalysator enthält Platin und Nickel auf Aktivkohle. Dabei wird zunächst Platin auf der Aktivkohle aufgebracht und reduziert und danach Nickel in Form eines Salzes auf den Träger aufgebracht. Das Nickel liegt bei diesem Katalysator als Hydroxid vor.

In EP 768 917 wird ein Katalysator zur Herstellung von Carbonsäure-Salzen beschrieben. Dieser besteht aus einem Ankermetall, beispielsweise Platin, das teilweise in einem alkaliresistenten Träger eingebettet ist und mindestens teilweise mit einem katalytisch wirkenden nichtedlen Metall, beispielsweise Nickel, durch stromloses Überziehen beschichtet wird. Bei diesem Katalysator liegen die beiden Metalle als getrennte Phasen auf dem Träger vor.

In US 4,185,036 wird ein Verfahren zur Hydrierung von Mischungen von Nitroaromaten beschrieben. Die verwendeten Katalysatoren enthalten Platin und gegebenenfalls ein weiteres Metall, beispielsweise Nickel, auf Aktivkohle. Das weitere Metall liegt dabei in Form des Oxids oder Hydroxids auf dem Träger vor.

DE 199 11 865 und DE 196 36 214 beschreiben Verfahren zur Hydrierung von Dinitrotoluol. Die eingesetzten Katalysatoren enthalten Iridium sowie mindestens ein Dotierungselement, beispielsweise Nickel oder Platin.

WO 03/39743 beschreibt ein Verfahren zur Herstellung von TDA unter Verwendung eines Hydrierkatalysators, bestehend aus Platin, einem weiteren Edelmetall und einem unedlen Metall.

In WO 05/037768 werden Katalysatoren und Verfahren zur Hydrierung von Dinitrotoluol zur Toluyldiamin beschrieben. Die Katalysatoren bestehen aus Platin und Nickel, wobei die beide Metalle auf dem Träger in Form einer Legierung vorliegen.

US 2004/0199017 wird ein Verfahren zur Hydrierung von Dinitrotoluol zur Toluyldiamin beschrieben, bei dem ein Katalysator verwendet wird, bei dem Nickel, Palladium und ein drittes Metall, ausgewählt aus der Gruppe, enthaltend Zink, Cadmium, Kupfer und Silber in einer Menge von 0,01 bis 10 Gew.-%, bezogen auf das Gewicht des Trägers, eingesetzt wird.

Ständige Aufgabe bei der Hydrierung von DNT zu TDA ist die weitere Erhöhung der Ausbeute und insbesondere die Verbesserung der Selektivität des Verfahrens, um so die Nebenreaktionen, die zur Bildung von hochmolekularen Nebenprodukten oder zur Bildung von Leichtsiedern führen, zurückzudrängen. Weiterhin sollte der Katalysator auch bei höheren Reaktionstemperaturen stabil sein und keine Verschlechterung der Selektivität des Verfahrens zulassen.

Um ein wirtschaftliches Verfahren zu betreiben, muss die Herstellung und die Aufarbeitung des Katalysators möglichst günstig sein. Die Herstellung des Katalysators wird billiger, wenn sie in möglichst wenigen Herstellungsschritten durchgeführt wird. Die Aufarbeitung von gebrauchten Edelmetallkatalysatoren wird billiger, wenn der Anteil an zusätzlichen Nichtedelmetall Komponenten möglichst niedrig wird.

Aufgabe der Erfindung war es demzufolge, Katalysatoren für die Hydrierung von aromatischen Nitroverbindungen zu den entsprechenden Aminen, insbesondere von DNT zu TDA, bereitzustellen, die zu einer höheren Ausbeute und Selektivität des Verfahrens führen und deren Herstellung sowie Aufarbeitung mit niedrigen Kosten verbunden ist.

Diese Aufgabe konnte überraschenderweise gelöst werden durch die Verwendung von Hydrierkatalysatoren, bestehend aus Nickel, Palladium und einem zusätzlichen Element, ausgewählt aus der Gruppe, enthaltend Kobalt, Eisen, Vanadium, Mangan, Chrom, Platin, Iridium, Gold, Bismut, Molybdän, Selen, Tellur, Zinn und Antimon auf einem Träger bei der Hydrierung von aromatischen Nitroverbindungen zu den entsprechenden Aminen.

Gegenstand der Erfindung ist somit ein Verfahren zur Herstellung von aromatischen Aminen durch katalytische Hydrierung der entsprechenden Nitroverbindungen, insbesondere zur Herstellung von Toluylendiamin durch Hydrierung von Dinitrotoluol, dadurch gekennzeichnet, dass Hydrierkatalysatoren eingesetzt werden, bei denen als aktive Komponente ein Gemisch von Nickel, Palladium und einem zusätzlichen Element, ausgewählt aus der Gruppe, enthaltend Kobalt, Eisen, Vanadium, Mangan, Chrom, Platin, Iridium, Gold, Bismut, Molybdän, Selen, Tellur, Zinn und Antimon auf einem Träger vorliegt.

Gegenstand der Erfindung sind weiterhin Katalysatoren für die Herstellung von aromatischen Aminen durch katalytische Hydrierung der entsprechenden Nitroverbindungen, insbesondere zur Herstellung von Toluylendiamin durch Hydrierung von Dinitrotoluol, dadurch gekennzeichnet, dass sie als aktive Komponente ein Gemisch von Nickel, Palladium und einem zusätzlichen Element, ausgewählt aus der Gruppe, enthaltend Kobalt, Eisen, Vanadium, Mangan, Chrom, Platin, Iridium, Gold, Bismut, Molybdän, Selen, Tellur, Zinn und Antimon auf einem Träger enthalten.

Gegenstand der Erfindung ist weiterhin die Verwendung von Hydrierkatalysatoren, enthaltend als aktive Komponente ein Gemisch von Nickel, Palladium und einem zusätzlichen Element, ausgewählt aus der Gruppe, enthaltend Kobalt, Eisen, Vanadium, Mangan, Chrom, Platin, Iridium, Gold, Wismut, Molybdän, Selen, Tellur, Zinn und Antimon auf einem Träger, zur Herstellung von aromatischen Aminen durch katalytische Hydrierung der entsprechenden Nitroverbindungen, insbesondere zur Herstellung von Toluylendiamin durch Hydrierung von Dinitrotoluol.

Das zusätzliche Element ist vorzugsweise ausgewählt aus der Gruppe, enthaltend Kobalt und Eisen.

Die Metallpartikel sind meist polykristallin und können mit einem hoch-auflösenden TEM (FEG-TEM: Field Emission Gun-Transmission Electron Microscopy) charakterisiert werden.

Als Träger für die Katalysatoren können die hierfür üblichen und bekannten Materialien eingesetzt werden. Vorzugsweise werden Aktivkohle, Ruß, Graphit oder Metalloxide, bevorzugt hydrothermal stabile Metalloxide wie z.B. ZrO₂ TiO₂ eingesetzt. Bei Graphit sind die HSAG (High Surface Area Graphite) mit einer Oberfläche von 50 bis 300 m²/g besonders bevorzugt. Besonders bevorzugt sind Aktivkohlen, insbesondere physikalisch oder chemisch aktivierte Aktivkohlen, oder Ruße, wie Acetylenruß.

Die erfindungsgemäßen Hydrierkatalysatoren enthalten vorzugsweise 5 bis 30 Gew.-%, insbesondere 10 bis 20 Gew.-% Nickel, 0,01 bis 20 Gew.-%, insbesondere 0,01 bis 5 Gew.-% Palladium und 0,1 bis 20 Gew.-%, insbesondere 0,01 bis 5 Gew.-% des zusätzlichen Elements, jeweils bezogen auf das Gewicht des Katalysators.

Bei der Durchführung des erfindungsgemäßen Hydrierverfahrens wird der erfindungsgemäße Katalysator vorzugsweise in einer Menge von 0,01 bis 10, besonders bevorzugt 0,01 bis 5 Gew.-%, insbesondere 0,2 bis 3 Gew.-%, bezogen auf das Reaktionsgemisch, eingesetzt.

Der Katalysator wird zumeist in reduziertem, vorzugsweise in reduziertem und passiviertem Zustand in den Reaktor eingebracht. Unter dem reduzierten und passivierten Zustand des Katalysators wird verstanden, dass der Katalysator nach der Herstellung aktiviert ist, danach jedoch, aus Sicherheitsgründen, die aktiven Zentren, beispielsweise durch Überleiten von Sauerstoff oder Kohlendioxid, passiviert werden. Alternativ kann der Katalysator unter einer inerten Atmosphäre oder in einem nicht leichtentzündlichen Lösungsmittel ausgebaut und stabilisiert werden, beispielsweise in Wasser, TDA/Wasser oder höheren Alkoholen, wie Butanol oder Ethylenglykol.

Das erfindungsgemäße Verfahren kann kontinuierlich oder diskontinuierlich unter Verwendung üblicher Reaktoren bei üblichen Verfahrensparametern, wie Druck und Temperatur, durchgeführt werden.

Vorzugsweise wird das Verfahren zur Herstellung von aromatischen Aminen, insbesondere TDA, unter Verwendung der erfindungsgemäßen Katalysatoren bei Drücken im Bereich von 5 bis 100 bar, besonders bevorzugt bei 10 bis 40 bar, insbesondere bei 20 bis 25 bar, durchgeführt.

Vorzugsweise wird das Verfahren zur Herstellung von aromatischen Aminen, insbesondere DNT, unter Verwendung der erfindungsgemäßen Katalysatoren bei einer Temperatur im Bereich von 80 bis 250 °C, besonders bevorzugt im Bereich von 100 bis 220 °C und insbesondere im Bereich 160 bis 200 °C durchgeführt.

Zumeist wird die Hydrierung in Form einer kontinuierlichen Suspensionshydrierung in üblichen und geeigneten Reaktoren durchgeführt. Als Reaktoren kommen beispielsweise Rührkessel oder Schlaufenreaktoren, wie zum Beispiel Strahlschlaufenreaktoren, sogenannte Loop-Venturi-Reaktoren, oder Schlaufenreaktoren mit innerer Strömungsumkehr, wie in WO 00/35852 beschrieben, zum Einsatz. Zur Abtrennung der Katalysatoren vom ausgeschleusten Reaktionsgemisch können beispielsweise Querstromfilter eingesetzt werden. Ein derartiges Verfahren ist beispielsweise in WO 03/66571 beschrieben.

Die bei der Hydrierung gebildeten Amine werden dem Hydriervorgang kontinuierlich oder diskontinuierlich entzogen und einer Aufarbeitung, beispielsweise einer destillativen Nachbehandlung, unterworfen.

Vorzugsweise werden im Rahmen des erfindungsgemäßen Verfahrens aromatische Nitroverbindungen mit einer oder mehreren Nitrogruppen und 6 bis 18 C-Atomen, bsp. Nitrobenzole, wie z.B. o-, m-, p-Nitrobenzol, 1,3-Dinitrobenzol, Nitrotoluole, wie z.B. 2,4-, 2,6-Dinitrotoluol, 2,4,6-Trinitrotoluol, Nitroxylole, wie z.B. 1,2-Dimethyl-3-, 1,2 Dimethyl-4-, 1,4-Dimethyl-2-, 1,3-Dimethyl-2-, 2,4-Dimethyl-1-und 1,3-Dimethyl-5-nitrobenzol, Nitronaphthaline, wie z.B. 1-, 2-Nitronaphthalin, 1,5 und 1,8-Dinitronaphthalin, Chlornitrobenzole, wie z.B. 2-Chlor-1,3-, 1-Chlor-2,4-dinitrobenzol, o-, m-, p-Chlornitrobenzol, 1,2-Dichlor-4-, 1,4-Dichlor-2-, 2,4-Dichlor-1-und 1,2-Dichlor-3-nitrobenzol, Chlornitrotoluole, wie z.B. 4-Chlor-2, 4-Chlor-3-, 2-Chlor-4- und 2-Chlor-6-nitrotoluol, Nitroaniline, wie z.B. o-, m-, p- Nitroanilin; Nitroalkohole, wie z.B. Tris(hydroxymethyl)nitromethan, 2-Nitro-2-methyl-, 2-Nitro-2-ethyl-1,3-propandiol, 2-Nitro-1-butanol und 2-Nitro-2-methyl-1-propanol sowie beliebige Gemische aus zwei oder mehreren der genannten Nitroverbindungen eingesetzt.

Bevorzugt werden nach dem erfindungsgemäßen Verfahren aromatische Nitroverbindungen, vorzugsweise Mononitrobenzol, Methylnitrobenzol oder Methylnitrotoluol und insbesondere 2,4-Dinitrotoluol oder dessen technische Gemische mit 2,6-Dinitrotoluol, wobei diese Gemische vorzugsweise bis zu 35 Gewichtsprozent, bezogen auf das Gesamtgemisch, an 2,6-Dinitrotoluol mit Anteilen von 1 bis 5 Prozent an vicinalem DNT und 0,5 bis 1,5 % an 2,5- und 3,5-Dinitrotoluol aufweisen, zu den entsprechenden Aminen hydriert.

Die erfindungsgemäßen Katalysatoren können bei einem Hydrierverfahren eingesetzt werden, bei dem die aromatische Nitroverbindung in reiner Form, als Mischung mit dem entsprechenden Di- und/oder Polyamin, als Mischung mit dem entsprechenden Di- und/oder Polyamin und Wasser, als Mischung mit dem entsprechenden Di- und/oder Polyamin, Wasser und einem alkoholischen Lösungsmittel oder als Mischung mit dem entsprechenden Di- und/oder Polyamin, Wasser, einem alkoholischen Lösungsmittel und einem katalysatorreaktivierenden Zusatz eingesetzt werden, wobei jeweils auch Gemische aus zwei oder mehr der oben genannten Nitroverbindungen, der entsprechenden Aminverbindungen, dem alkoholischen Lösungsmittel und dem katalysatorreaktivierenden Zusatz eingesetzt werden können.

Sofern ein oben beschriebenes Gemisch eingesetzt wird, liegt das Verhältnis von Aminverbindung zu Wasser vorzugsweise im Bereich von 10:1 bis 1:10, besonders bevorzugt im Bereich von 4:1 bis 1:1 und das Verhältnis des Amin/Wasser-Gemischs zu mindestens einem alkoholischen Lösungsmittel vorzugsweise bei 1000:1 bis 1:1, besonders bevorzugt bei 50:1 bis 5:1.

Um Nebenreaktionen zu unterdrücken, ist es bevorzugt, das Verfahren so zu führen, dass der Katalysator an seiner Belastungsgrenze gefahren wird. Dies kann beispielsweise durch die Menge der zudosierten Nitroverbindung, die Menge des Katalysators im Reaktionsgemisch, die Temperatur oder den Druck gesteuert werden.

Unter der Belastungsgrenze des Katalysators wird die Menge der hydrierbaren Stickstoff- und Sauerstoffatome enthaltenden Gruppen verstanden, die vom Katalysator bei gegebenen Druck- und Temperaturbedingungen hydriert werden können. Bei den Stickstoff- und Sauerstoffatome enthaltenden Gruppen kann es sich neben Nitrogruppen auch um Nitrosogruppen und Nitrosamingruppen handeln.

Die Herstellung der erfindungsgemäßen Katalysatoren erfolgt beispielsweise, indem der Träger vorgelegt und mit einer wässrigen Lösung der Palladium- und Nickelsalze zusammen mit dem Zusatzelement gebracht wird. Dabei sollte die Menge des zur Lösung der Salze verwendeten Wassers so bemessen sein, dass eine knetbare Paste entsteht. Vorzugsweise wird das Wasser in einer Menge von 100 bis 200 Gew.-% der Trägermasse eingesetzt. Als Metallsalze kommen insbesondere Nitrate oder Chloride zum Einsatz, wobei Nitrate wegen ihrer geringeren Korrosivität bevorzugt sind. Die Paste wird gemischt und danach das Wasser bei geringem Druck und Temperaturen im Bereich zwischen 50 und 100 °C verdampft, beispielsweise in einem Rotationsverdampfer oder einem Ofen. Aus Sicherheitsgründen kann das Verdampfen in einem Stickstoffstrom erfolgen. Die Fixierung der Metalle auf dem Träger kann bei der Verwendung von Chloriden als Metallsalze durch Reduktion mit Wasserstoff erfolgen. Hierbei kann jedoch Korrosion auftreten. Bevorzugt werden die Metalle daher alkalisch fixiert. Dies erfolgt insbesondere durch Zugabe einer wässrigen Lösung von Alkalicarbonaten und nachfolgendes anionfrei waschen des Trägers. Alternativ können die Metalle auch alkalisch, insbesondere bei einem pH-Wert im Bereich von 8 bis 9, aus einer überstehenden Lösung auf den Träger gefällt werden. Danach wird der Träger getrocknet, vorzugsweise wie oben beschrieben, und mit Wasserstoff reduziert. Dies kann beispielsweise in einem Drehkugelofen geschehen. Vor dem Ausbau des Katalysators wird dieser passiviert, beispielsweise unter einem Inertgas, wie Stickstoff, der Spuren von Luft, vorzugsweise nicht mehr als 10 Vol.-%, enthält.

Die nach diesem Verfahren hergestellten erfindungsgemäßen Hydrierkatalysatoren enthalten vorzugsweise 0,5 bis 5 Gew.-% Palladium, 10 bis 20 Gew.-% Nickel und 0,5 bis 5 Gew.-% des Zusatzelementes.

In einer anderen Ausführungsform der Herstellung der erfindungsgemäßen Hydrierkatalysatoren erfolgt die Reduktion der Katalysatoren durch Zusatz von reduzierend wirkenden Salzen, wie Ammoniumcarboxylaten oder Alkalicarboxylaten, beispielsweise Ammoniumformiat oder Natriumformiat. Dazu wird der Träger mit Wasser suspendiert und gleichzeitig oder nach der Suspendierung die Lösungen der Metallsalze zugegeben. Als Metallsalze kommen insbesondere Nitrate oder Chloride zum Einsatz, wobei Nitrate wegen ihrer geringeren Korrosivität bevorzugt sind. Zu dieser Lösung werden die reduzierend wirkenden Salze zugegeben und die Suspension erhitzt, beispielsweise durch Kochen unter Rückfluss. Anschließend werden die Katalysatoren anionenfrei gewaschen und filtriert, beispielsweise durch eine Filterpresse oder eine Zentrifuge, und als feuchte Paste verwendet.

Die nach diesem Verfahren hergestellten erfindungsgemäßen Hydrierkatalysatoren enthalten vorzugsweise 0,5 bis 5 Gew.-% Palladium, 10 bis 20 Gew.-% Nickel und 0,5 bis 5 Gew.-% des Zusatzelementes.

Durch die Verwendung der erfindungsgemäßen Katalysatoren ist es möglich, die Hydrierung von DNT zu TDA bei Temperaturen im Bereich zwischen 120 und 250°C, insbesondere 120 bis 200°C, bei denen sich bei Verwendung herkömmlicher Katalysatoren die Selektivität der Umsetzung stark verschlechtert, durchzuführen. Eine Erhöhung der Reaktionstemperatur ist vorteilhaft da die Löslichkeiten der einzelnen Komponenten höher sind, und auch die Reaktionsgeschwindigkeit mit der Temperatur zunimmt. Man kann also die RZA (Raum Zeit Ausbeute) erhöhen, solange die Reaktionsenergie sicher abgeführt werden kann.

Die Erfindung soll an den nachstehenden Beispielen näher erläutert werden.

Beispiel 1 (Vergleich):

Ein Aktivkohleträger Norit® SX+ Träger wurde in Wasser zu einer 10 %-igen Suspension suspendiert. Dazu wurden Pd(II)-Nitrat für 1,0 Gew.-% Palladium, bezogen auf das Gewicht des Katalysators, Ni(II)Nitrat-Hexahydrat für 15 Gew.-% Nickel und Zink(II)-Nitrat Hexahydrat für 1,0 Gew.-% Zink gegeben und mit Ammoniumformiat unter Rückfluss für 2 Stunden gekocht. Der so erhaltene Katalysator wurde nitratfrei gewaschen. Der so erhaltene Katalysator wird als Katalysator 1 bezeichnet.

Der so erhaltene Katalysator hatte einen Gehalt von 0,92 Gew.-% Palladium, 14 Gew.-% Nickel und 0,96 Gew.-% Zink.

### Beispiel 2:

Es wurde verfahren wie in Beispiel 1, es wurde jedoch 1,0 Gew.-% Palladium, 15 Gew.-% Nickel und 1,0 Gew.-% Zinn zugesetzt. Der so erhaltene Katalysator wird als Katalysator 2 bezeichnet. Der so erhaltene Katalysator hatte einen Gehalt von 0,80 Gew.-% Palladium, 13 Gew.-% Nickel und 0,93 Gew.-% Zinn.

### Beispiel 3:

Es wurde verfahren wie in Beispiel 1, es wurde jedoch 1,0 Gew.-% Palladium, 15 Gew.-% Nickel und 1,0 Gew.-% Gold zugesetzt. Der so erhaltene Katalysator wird als Katalysator 3 bezeichnet. Der so erhaltene Katalysator hatte einen Gehalt von 0,98 Gew.-% Palladium, 10 Gew.-% Nickel und 0,96 Gew.-% Gold.

### Beispiel 4:

Es wurde verfahren wie in Beispiel 1, es wurde jedoch 1,0 Gew.-% Palladium, 15 Gew.-% Nickel und 1,0 Gew.-% Eisen zugesetzt. Der so erhaltene Katalysator wird als Katalysator 4 bezeichnet. Der so erhaltene Katalysator hatte einen Gehalt von 0,74 Gew.-% Palladium, 8 Gew.-% Nickel und 0,83 Gew.-% Eisen.

### Beispiel 5:

Es wurde verfahren wie in Beispiel 1, es wurde jedoch 1,0 Gew.-% Palladium, 15 Gew.-% Nickel und 1,0 Gew.-% Kobalt zugesetzt. Der so erhaltene Katalysator wird als Katalysator 5 bezeichnet. Der so erhaltene Katalysator hatte einen Gehalt von 0,85 Gew.-% Palladium, 13 Gew.-% Nickel und 0,81 Gew.-% Kobalt.

### Hydrierung von DNT zu TDA

Die Hydrierung von DNT zu TDA, wurde in einem kontinuierlichen 300 ml Rührkessel durchgeführt, der Katalysator wurde mechanisch im Reaktor zurückgehalten.

Der Katalysator wurde im Wasser suspendiert und in den Reaktor gebracht (Katalysatormenge 1 bis 2 Gew.-% des Flüssigkeitsvolumens des Reaktors), und auf eine Temperatur von 180°C gebracht. Unter einem Wasserstoff-Druck von 25 bar wurde DNT in einer solchen Menge kontinuierlich als Schmelze zudosiert, dass eine Raum-ZeitAusbeute von 150-600 Kg_{TDA}/m³.h eingestellt wurde. Proben wurden mittels Gaschromatographie analysiert: Die TDA Ausbeute, Bildung von Hochsiedern und Leichtsiedern wurde verfolgt.

Die Katalysatoren, deren Zusammensetzung sowie die Ergebnisse sind der Tabelle 1 zu entnehmen.

| Beispiel | KAT | | TDA |
|---|---|---|---|
| # | | | Sel. % |
| 1 | 0,92%Pd-14%Ni-0,96Zn/C | Ca. 200 | 98,77 |
| (vergleich) | | Ca 700 | 98,97 |
| 2 | 0,80% Pd-13% N i-0,93Sn/C | Ca 400 | 98,47 |
| | | Ca 700 | 98,51 |
| 3 | 0,98% Pd-10% Ni-0,96Au/C | Ca 200 | 98,62 |
| | | Ca 700 | 98,83 |
| 4 | 0,74%Pd-8%Ni-0,83Fe/C | Ca 400 | 98,79 |
| | | Ca 700 | 99,04 |
| 5 | 0,85%Pd-13%Ni-0,81Co/C | Ca 400 | 98,82 |
| | | Ca 700 | 99,07 |

Die Beispiele zeigen, dass die erfindungsgemäßen Katalysatoren zu einer hohen Selektivität des Verfahrens führen. Insbesondere bei der Verwendung von Eisen und Kobalt werden sehr gute Ergebnisse erzielt.

## Patentansprüche

1. Verfahren zur Herstellung von aromatischen Aminen durch katalytische Hydrierung der entsprechenden Nitroverbindungen, insbesondere zur Herstellung von Toluylendiamin durch Hydrierung von Dinitrotoluol, **dadurch gekennzeichnet, dass** Hydrierkatalysatoren eingesetzt werden, bei denen als aktive Komponente ein Gemisch von Nickel, Palladium und einem zusätzlichen Element, ausgewählt aus der Gruppe, enthaltend Kobalt, Eisen, Vanadium, Mangan, Chrom, Platin, Iridium, Gold, Bismut, Molybdän, Selen, Tellur, Zinn und Antimon auf einem Träger vorliegt, wobei der Träger ausgewählt ist aus der Gruppe, enthaltend Aktivkohle, Ruß, Graphit und Metalloxide, und der Katalysator 5 bis 30 Gew.-% Nickel, 0,01 bis 20 Gew.% Palladium und 0,01 -20 Gew.% des zusätzlichen Elements, jeweils bezogen auf das Gewicht des Katalysators, enthält.

2. Verfahren nach Anspruch 1, **dadurch gekennzeichnet, dass** das zusätzliche Metall ausgewählt ist aus der Gruppe, enthaltend Kobalt und Eisen.

3. Verfahren nach Anspruch 1, **dadurch gekennzeichnet, dass** der Katalysator 10 bis 20 Gew.% Nickel, 0,5 bis 5 Gew.-% Palladium und 0,5 - 5 Gew.% des zusätzlichen Elements, jeweils bezogen auf das Gewicht des Katalysators, enthält.

4. Verfahren nach Anspruch 1, **dadurch gekennzeichnet, dass** der Katalysator in einer Menge von 0,01 bis 5 Gew.-%, bezogen auf die Reaktionsmischung, eingesetzt wird.

5. Katalysatoren zur Herstellung von aromatischen Aminen durch katalytische Hydrierung der entsprechenden Nitroverbindungen, insbesondere zur Herstellung von Toluylendiamin durch Hydrierung von Dinitrotoluol, **dadurch gekennzeichnet, dass** die Hydrierkatalysatoren als aktive Komponente ein Gemisch von Nickel, Palladium und einem zusätzlichen Element, ausgewählt aus der Gruppe, enthaltend Kobalt, Eisen, Vanadium, Mangan, Chrom, Platin, Iridium, Gold, Bismut, Molybdän, Selen, Tellur, Zinn und Antimon auf einem Träger enthalten, wobei der Träger ausgewählt ist aus der Gruppe, enthaltend Aktivkohle, Ruß, Graphit und Metalloxide, und der Katalysator 5 bis 30 Gew.-% Nickel, 0,01 bis 20 Gew.-% Palladium und 0,01 - 20 Gew.-% des zusätzlichen Elements, jeweils bezogen auf das Gewicht des Katalysators, enthält.

6. Katalysatoren nach Anspruch 5, **dadurch gekennzeichnet, dass** das zusätzliche Element ausgewählt ist aus der Gruppe, enthaltend Kobalt und Eisen.

7. Katalysatoren nach Anspruch 5, **dadurch gekennzeichnet, dass** der Katalysator 10 bis 20 Gew.% Nickel, 0,5 bis 20 Gew.-% Palladium und 0,5 - 5 Gew.% des zusätzlichen Elements, jeweils bezogen auf das Gewicht des Katalysators, enthält.

## Claims

1. A process for preparing aromatic amines by catalytic hydrogenation of the corresponding nitro compounds, in particular for preparing toluenediamine by hydrogenation of dinitrotoluene, wherein hydrogenation catalysts in which a mixture of nickel, palladium and an additional element selected from the group consisting of cobalt, iron, vanadium, manganese, chromium, platinum, iridium, gold, bismuth, molybdenum, selenium, tellurium, tin and antimony is present as active component on a support are used, the support being selected from the group consisting of activated carbon, carbon black, graphite and metal oxides, and the catalyst comprising from 5 to 30% by weight of nickel, from 0.01 to 20% by weight of palladium and 0.01-20% by weight of the additional element, in each case based on the weight of the catalyst.

2. The process according to Claim 1, wherein the additional metal is selected from the group consisting of cobalt and iron.

3. The process according to Claim 1, wherein the catalyst comprises from 10 to 20% by weight of nickel, from 0.5 to 5% by weight of palladium and 0.5-5% by weight of the additional element, in each case based on the weight of the catalyst.

4. The process according to Claim 1, wherein the catalyst is used in an amount of from 0.01 to 5% by weight, based on the reaction mixture.

5. A catalyst for the preparation of aromatic amines by catalytic hydrogenation of the corresponding nitro compounds, in particular for the preparation of toluenediamine by hydrogenation of dinitrotoluene, which comprises a mixture of nickel, palladium and an additional element selected from the group consisting of cobalt, iron, vanadium, manganese, chromium, platinum, iridium, gold, bismuth, molybdenum, selenium, tellurium, tin and antimony as active component on a support, the support being selected from the group consisting of activated carbon, carbon black, graphite and metal oxides, and the catalyst comprising from 5 to 30% by weight of nickel, from 0.01 to 20% by weight of palladium and 0.01-20% by weight of the additional element, in each case based on the weight of the catalyst.

6. The catalyst according to Claim 5, wherein the additional element is selected from the group consisting of cobalt and iron.

7. The catalyst according to claim 5 which comprises from 10 to 20% by weight of nickel, from 0.5 to 20% by weight of palladium and 0.5-5% by weight of the additional element, in each case based on the weight of the catalyst.

## Revendications

1. Procédé pour la préparation d'amines aromatiques par hydrogénation catalytique des composés nitro correspondants, en particulier pour la préparation de toluylènediamine par hydrogénation de dinitrotoluène, **caractérisé en ce qu'**on utilise des catalyseurs d'hydrogénation, dans lesquels se trouve, comme composant actif, un mélange de nickel, de palladium et d'un élément supplémentaire choisi dans le groupe contenant le cobalt, le fer, le vanadium, le manganèse, le chrome, le platine, l'iridium, l'or, le bismuth, le molybdène, le sélénium, le tellure, l'étain et l'antimoine sur un support, le support étant choisi dans le groupe contenant le charbon actif, la suie, le graphite et les oxydes métalliques, et le catalyseur contenant 5 à 30% en poids de nickel, 0,01 à 20% en poids de palladium et 0,01-20% en poids de l'élément supplémentaire, à chaque fois par rapport au poids du catalyseur.

2. Procédé selon la revendication 1, **caractérisé en ce que** le métal supplémentaire est choisi dans le groupe contenant le cobalt et le fer.

3. Procédé selon la revendication 1, **caractérisé en ce que** le catalyseur contient 10 à 20% en poids de nickel, 0,5 à 5% en poids de palladium et 0,5-5% en poids de l'élément supplémentaire, à chaque fois par rapport au poids du catalyseur.

4. Procédé selon la revendication 1, **caractérisé en ce que** le catalyseur est utilisé en une quantité de 0,01 à 5% en poids, par rapport au mélange réactionnel.

5. Catalyseurs pour la préparation d'amines aromatiques par hydrogénation catalytique des composés nitro correspondants, en particulier pour la préparation de toluylènediamine par hydrogénation de dinitrotoluène, **caractérisés en ce que** les catalyseurs d'hydrogénation contiennent, comme composant actif, un mélange de nickel, de palladium et d'un élément supplémentaire choisi dans le groupe contenant le cobalt, le fer, le vanadium, le manganèse, le chrome, le platine, l'iridium, l'or, le bismuth, le molybdène, le sélénium, le tellure, l'étain et l'antimoine sur un support, le support étant choisi dans le groupe contenant le charbon actif, la suie, le graphite et des oxydes métalliques, et le catalyseur contenant 5 à 30% en poids de nickel, 0,01 à 20% en poids de palladium et 0,01-20% en poids de l'élément supplémentaire, à chaque fois par rapport au poids du catalyseur.

6. Catalyseurs selon la revendication 5, **caractérisés en ce que** l'élément supplémentaire est choisi dans le groupe contenant le cobalt et le fer.

7. Catalyseurs selon la revendication 5, **caractérisés en ce que** le catalyseur contient 10 à 20% en poids de nickel, 0,5 à 20% en poids de palladium et 0,5-5% en poids de l'élément supplémentaire, à chaque fois par rapport au poids du catalyseur.
